(19) Europäisches Patentamt
European Patent Office
Office européen des brevets

(11) Publication number: 0 281 018 A1

(12) # EUROPEAN PATENT APPLICATION

(21) Application number: 88102814.6

(22) Date of filing: 25.02.88

(51) Int. Cl.[4]: C07D 333/20 , C07D 307/52 , A61K 31/38 , A61K 31/34

(30) Priority: 26.02.87 US 19103

(43) Date of publication of application: 07.09.88 Bulletin 88/36

(84) Designated Contracting States: AT BE CH DE ES FR GB GR IT LI LU NL SE

(71) Applicant: MERRELL DOW PHARMACEUTICALS INC.
2110 E. Galbraith Road
Cincinnati Ohio 45215(US)

(72) Inventor: Bargar, Thomas M.
125 Lynn Court
Zionsville Indiana 46077(US)
Inventor: McCarthy, James R.
4907 David Court
Cincinnati Ohio 45215(US)
Inventor: Creemer, Lawrence C.
7248 Causeway Drive, Apt. 2A
Indianapolis Indiana 46214(US)

(74) Representative: Vossius & Partner
Siebertstrasse 4 P.O. Box 86 07 67
D-8000 München 86(DE)

(54) **Heterocyclyl-2-propyn-1-amines.**

(57) This invention relates to novel heterocyclyl-2-propyn-1-amines which are useful as dopamine beta hydroxylase inhibitors in the treatment of hypertension.

EP 0 281 018 A1

## HETEROCYCLYL-2-PROPYN-1-AMINES

This invention relates to novel heterocyclyl-2-propyn-1-amines, to intermediates and processes for their production and to pharmaceutical compositions for treating hypertension containing such compounds.

More specifically, this invention relates to propynyl amines of the general formula

$$Het\text{-}C\equiv C\text{-}CH_2\text{-}NH_2 \quad (I)$$

wherein Het is 2-or 3-thienyl or 2-or 3-furyl, each of the Het thienyl and furyl rings optionally being substituted with from 1 to 3 moieties selected from the group consisting of lower alkyl of from 1 to about 6 carbon atoms, alkoxy of from 1 to about 6 carbon atoms, alkylthio of from 1 to about 6 carbon atoms, carboxylic acid, halo or hydroxymethyl and the therapeutically acceptable acid addition salts thereof. The compounds are inhibitors of dopamine beta hydroxylase, and are useful as antihypertensive agents.

When thienyl or furyl heterocyclic rings are di-or trisubstituted, the substituents can be the same or they can be different. Preferably, the heterocycle is either unsubstituted or is monosubstituted.

As used herein, the term "lower alkyl" means and includes groups from one to about six carbon atoms and include methyl, ethyl, propyl, butyl, pentyl and hexyl groups, which can be straight-or branched-chain groups. The term "alkoxy" refers to -OR groups wherein R is defined as lower alkyl above. The term "alkylthio" refers to -SR groups wherein R is defined as lower alkyl above. The term "halo" refers to chloro, bromo and fluoro. The preferred substituents include methyl; methoxy; methylthio and halo.

Illustrative examples of compounds of this invention include 3-(2-thienyl)-2-propyn-1-amine; 3-(3-thienyl)-2-propyn-1-amine; 3-(2-furyl)-2-propyn-1-amine; 3-(3-furyl)-2-propyn-1-amine; 3-[2-(5-chlorothienyl)]-2-propyn-1-amine; 3-[2-(5-methylthienyl)]-2-propyn-1-amine; 3-[2-(5-fluorothienyl)]-2-propyn-1-amine; 3-[3-(5-methylthiothienyl)]-2-propyn-1-amine; 3-[3-(5-hydromethylthienyl)]-2-propyn-1-amine; 3-[2-(5-methylfuryl)-]-2-propyn-1-amine; 3-[3-(5-fluorofuryl)-2-propyn-1-amine; 3-[3-(5-carboxyfuryl)]-2-propyn-1-amine and the therapeutically acceptable acid addition salts thereof.

Representative salts are those salts formed with non-toxic organic or inorganic acids, such as, for example, those formed from the following acids: hydrochloric, hydrobromic, sulfonic, sulfuric, phosphoric, nitric, maleic, fumaric, benzoic, ascorbic, succinic, methanesulfonic, acetic, propionic, tartiaric, citric, lactic, malic, mandelic, cinnamic, palmitic, itaconic, benzenesulfonic and toluenesulfonic.

The propynylamines (I) of this invention can readily be prepared by a series of reactions illustrated by the following reaction scheme:

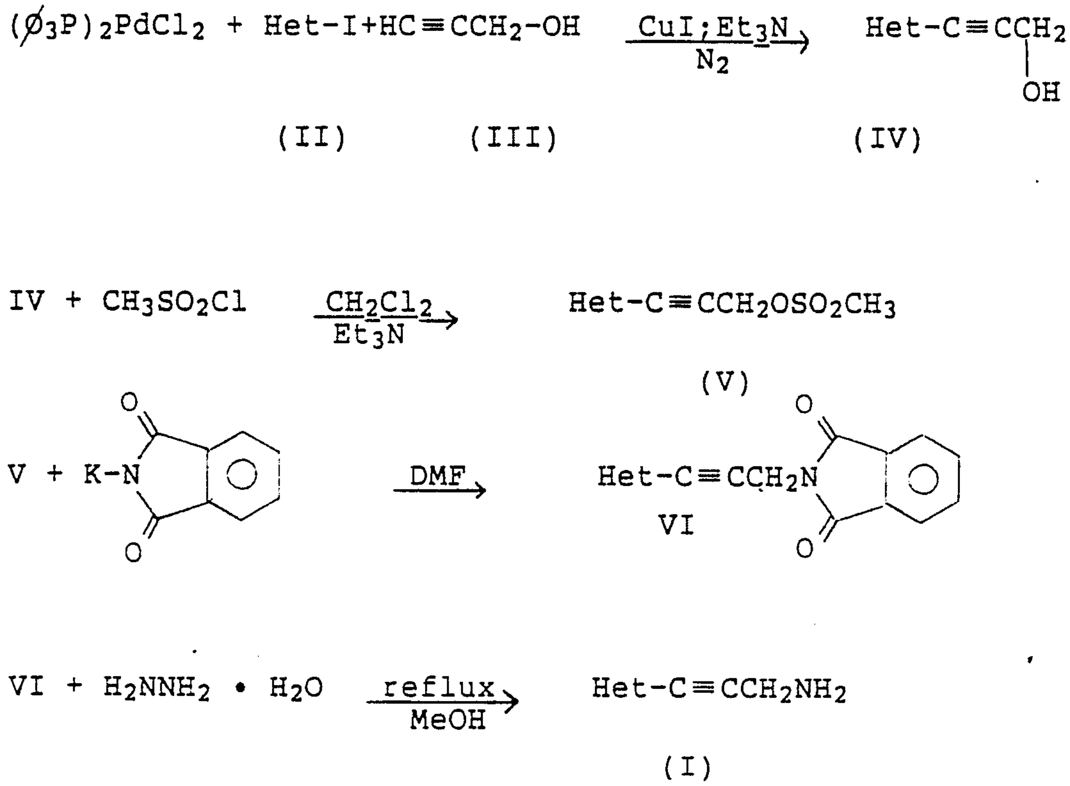


(II) (III) (IV) (V) VI (I)

wherein φ represents phenyl, and Het is as previously defined.

In essence, the foregoing reaction scheme shows that the propynylamines of group (I) can be prepared by reacting the appropriate 2-propyn-1-yl phthalimide (VI) with hydrazine hydrate ($H_2NNH_2 \bullet H_2O$) in an organic solvent such as methanol (MeOH) under reflux conditions. The appropriate phthalimide (VI) can be obtained by known methodology, one route of which is presented in the reaction scheme above, wherein 2-propyn-1-ol (III) is reacted with an appropriate 2-or 3-iodide-substituted heterocycle (II) wherein Het is as described above, in the presence of catalysts such as bis(triphenylphosphine)palladium (II) chloride [$(\phi_3 3P)_2PdCl_2$] and copper iodide (CuI), and triethylamine ($EtN_3$) under nitrogen to produce the alcohols depicted by (IV). The appropriate alcohol can then be combined with methanesulfonyl chloride ($CH_3SO_2Cl$) in the presence of dichloromethane ($CH_2Cl_2$)and $Et_3N$ to form the appropriate mesylate (V) and the mesylate can then be reacted with potassium phthalimide (K-phthalimide) in N,N-dimethylformamide (DFM) to produce (VI). The free bases of the compounds in group (I) can be converted to acid addition salts by conventional methodology.

The foregoing reaction scheme is further illustrated by the following specific example:

## EXAMPLE 1

### 3-(2-thienyl)-2-propyn-1-amine

A mixture of CuI (5.96 grams (g); 0.0313 moles (m)), bis(triphenylphosphine)palladium chloride (11.0 g; 0.0157 m) and 800 ml triethylamine was added *via* syringe to a solution (under nitrogen) of 2-propyn-1-ol (27.2 g; 0.485 mole) in 50 ml triethylamine. To the resulting brown suspension was added a solution of 2-iodothiophene (84.9 g 0.404 mole) in 50 ml triethylamine. The solution was added thereto slowly *via* syringe. The reaction mixture was allowed to reach 40°C before being cooled in an ice bath to 20°C. After 30 min., the mixture was filtered and the filtrate was concentrated at reduced pressure to yield a dark oil. Purification by bulb-to-bulb distillation (120°C/0.4 torr) yielded 49.6 g (89%) of the alcohol as yellow oil.

The alcohol (10.0 g, 0.0725 mole) was mixed with triethylamine (8.07 g 0.0797 mole) and the resulting mixture was added dropwise to a solution of 9.1 g (0.0794 mole) methanesulfonyl chloride in 100 ml dichloromethane at 0°C under nitrogen ($N_2$). The temperature during the addition was kept below 15°C. After 15 min. from the completion of the first addition, an additional 2.0 ml of methanesulfonyl chloride were added to the mixture. After 10 min., the reaction mixture was partitioned between dichloromethane and 1N

HCl. The dichloromethane layer was washed with saturated $NaHCO_3$ solution, dried over $K_2CO_3$, and concentrated at 25°C in vacuo to give the unstable oily mesylate.

The crude mesylate thus obtained was immediately taken up in 200 ml DMF, potassium phthalimide (14.44 g, (0.078 mole) was added, and the mixture was stirred at 25-30°C for 45 min. Completion of the reaction was judged by tracking the presence of starting material by tlc (20% ethylacetate/hexane). The reaction mixture was partitioned between water and dichloromethane, and the dichloromethane layer was dried over $K_2CO_3$ and concentrated to a brown semi-solid. This material was dissolved in ether, washed with water, decolorized again with carbon, dried over $K_2CO_3$, and filtered and concentrated. The resulting pale yellow solid was recrystallized from hexane/ethyl acetate to provide a total of 3.89 g (20%) phthalimide.

The phthalimide (3.72 g, 13.9 mmol) was suspended in 40 ml methanol, hydrazine hydrate (0.76 ml, 15.7 mmol) was added, and the mixture was warmed to reflux. The reaction was judged complete by tlc monitoring (25% hexane/chloroform), the cooled mixture was partitioned between 1N KOH and ether. The ether layer was washed with saturated NaCl solution, dried over $K_2CO_3$, and concentrated to a pale yellow oil which was purified by bulb-to-bulb distillation (160°C/1.0 torr) yielding 1.46 g (76%) free base. $^1$H-nmr (60 MHz, $CDCl_3$) 6.8-7.3 (complex pattern, 3H, thienyl protons), 3.59 (S, 2H, $CH_2$), 1.40 (S, 2H, $NH_2$). The free base was taken up in ether and treated with ethereal HCL to precipitate the hydrochloride. The ether was removed in vacuo at 25°C and the residue was recrystallized from 2-propanol, providing 1.82 g of the desired title product as colorless crystals with a mp of 199-200°C. Anal Calcd for $C_7H_7NS \bullet HCl$: C, 48.41; H, 4.64; N, 8.07; Found: C, 48.37; H, 4.53; N, 8.14.

The compounds of this invention exert in vitro and in vivo pharmacological effects as they inhibit dopamine-beta-hydroxylase (DBH) and, as such, are useful in the treatment of hypertension. An embodiment of this invention thus includes a method of treating hypertension in a mammal in need thereof which comprises administering internally to said animal an effective antihypertensive amount of a compound of formula I. Since DBH is a major enzyme in the synthetic pathway of norepinephrine (NE), it would be expected that the presence of an inhibitor would act to decrease the amount of NE produced, and thereby have an antihypertensive effect.

The DBH inhibitory properties of the compounds of this invention can readily be determined by standard and well-known procedures. For instance, determination of whether DBH inhibition demonstrates time-dependent kinetics is exemplified by a procedure wherein enzymatic oxygenation by DBH is determined in aqueous solution in the presence of molecular oxygen, an electron donor such as ascorbate, and the necessary cofactors for the enzyme at a pH of 4.5 to 5.5, preferably pH 5.0, and at a temperature of 20°C to 40°C, preferably 37°C. The test compound is added at the desired concentration, and the system is incubated. At different time internals, aliquots are taken and DBH activity is measured using tyramine as the substrate. The reaction is followed by measuring oxygen uptake using a polarographic electrode and an oxygen monitor by the method of S. May et al., J. Bio. Chem. 256, 2258 (1981). In tests utilizing the above described procedure, the DBH inhibitory activity of the test compound increased as a function of the time of incubation, as indicated in Table I.

TABLE I

TIME-DEPENDENT

DBH INHIBITORY ACTIVITY - IN VITRO

| Compound | Concentration (M) | t 1/2 |
|---|---|---|
| 3-(2-thienyl)-2-propyn-1-amine | $5 \times 10^{-5}$ | 9.2 min. |

t 1/2: time required for 50% log activity remaining

The ability of the compounds of this invention to lower blood pressure can be determined in vivo using standard and well-known procedures such as those employed for the continuous recording of arterial blood

pressure in conscious animals. For instance, test compounds are administered intraperitoneally (ip) to unanesthetized spontaneously hypertensive rats and the arterial blood pressure is monitored continuously. In tests utilizing the above procedure, the antihypertensive effect of the test compound is readily apparent as indicated by the degree of lowering of mean blood pressure (MBP) noted in Table II.

TABLE II

DBH INHIBITORY ACTIVITY - IN VIVO

| Compound | Dose mg/kg | Max Change MBP +/St. dev. | Duration hours |
|---|---|---|---|
| 3-(2-thienyl)-2-propyn-1-amine | 30 (ip) | -20+/-8 mm Hg | 14 |

Thus, based upon these and other standard laboratory techniques used to evaluate DBH inhibitors by standard toxicity tests and by standard pharmacological assays for the determination of antihypertensive activity in mammals, and by comparison of these results with the results of known antihypertensive agents, the effective antihypertensive dosage of the compounds of this invention can readily be determined. In general, effective antihypertensive results can be achieved at a dose of about 5 to about 100 mg per kilogram body weight per day. Of course, the specific initial and continuing dosage regimen for each patient will vary according to the nature and severity of the hypertension as determined by the attending diagnostician.

In their function as therapeutically useful compounds, it is advantageous to administer antihypertensive amounts of the compounds of this invention to the host animal as compositions in admixture with a pharmaceutical carrier suitable for enteral or parenteral administration. Such compositions may be in such forms as, for example, tablets, capsules and suppositories, or in liquid forms, as for example, elixirs, emulsions, sprays and injectables. In the formulation of pharmaceutical compositions, one skilled in the art can readily select those substances which do not react with the compounds of this invention, such as. for example, water, gelatin, lactose, starches, magnesium sterate, talc, vegetable oils, benzyl alcohols, gums, polyalkylene glycols, petroleum jelly and the like. The active ingredient is preferably present in the composition in such proportions by weight that the proportion by weight of the active ingredient to be administered lies between 0.1% and 50%.

## Claims

1. Propynyl amines of the formula I

$$Het\text{-}C{\equiv}C\text{-}CH_2\text{-}NH_2 \qquad (I)$$

wherein Het is 2-or 3-thienyl or 2-or 3-furyl, each Het ring optionally being substituted with from 1 to 3 moieties selected from lower alkyl of from one to about six carbon atoms, alkoxy of from 1 to about 6 carbon atoms, alkylthio of from 1 to about 6 carbon atoms, fluoro, bromo, chloro, carboxylic acid and hydroxymethyl and the pharmaceutically acceptable acid addition salts thereof.

2. A compound of claim 1 wherein Het is 2-or 3-thienyl.
3. A compound of claim 1 wherein Het is 2-or 3-furyl.
4. A compound of claim 2 wherein Het is 2-thienyl.
5. A compound of claim 2 wherein Het is 3-thienyl.
6. A compound of claim 3 wherein Het is 2-furyl.
7. A compound of claim 3 wherein Het is 3-furyl.
8. A compound of claim 1 wherein Het is substituted 2-or 3-thienyl.
9 . A compound of claim 1 wherein Het is substituted 2-or 3-furyl.

10. A compound of claim 8 or 9 wherein Het is monosubstituted with chloro, bromo, fluoro, methyl or methylthio.

11. A compound of claim 8 or 9 wherein the monosubstitution is at the 5 position of the Het ring.

12. A compound of claim 8 or 9 wherein the monosubstitution is chloro, fluoro or bromo.

13. 3-(2-thienyl)-2-propyn-1-amine.

14. A process for preparing a compound of the formula I

$Het\text{-}C{\equiv}C\text{-}CH_2\text{-}NH_2$ (I)

wherein Het is 2-or 3-thienyl or 2-or 3-furyl, each Het ring optionally being substituted with from 1 to 3 moieties selected from the group consisting of lower alkyl of from one to about six carbon atoms, alkoxy of from 1 to about 6 carbon atoms, alkylthio of from 1 to about 6 carbon atoms, fluoro, bromo, chloro, carboxylic acid and hydroxymethyl and the pharmaceutically acceptable acid addition salts thereof which comprises reacting a compound of the formula

$Het\text{-}C{\equiv}CCH_2OSO_2CH_3$

with potassium phthalimide and then treating the resulting phthalimide protected amine group with a deprotecting agent.

15. A propynyl amine according to anyone of claims 1 to 13 for use as a pharmaceutically active compound.

16. A propynyl amine according to anyone of claims 1 to 13 for use as a pharmaceutically active compound for the treatment of hypertension in mammals.

17. A pharmaceutical composition comprising a propynyl amine according to anyone of claims 1 to 13 and optionally a pharmaceutically acceptable carrier.

18. A pharmaceutical composition for treating hypertension in mammals which comprises an effective amount of a propynyl amine according to anyone of claims 1 to 13 and optionally a pharmaceutically acceptable carrier.

European Patent Office

# EUROPEAN SEARCH REPORT

Application Number

EP 88 10 2814

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 104, no. 15, 14th April 1986, page 684, abstract no. 129726m, Columbus, Ohio, US; T.M. BARGAR et al.: "Unsaturated heterocyclic amines as potent time-dependent inhibitors of dopamine beta-hydroxylase", J. MED. CHEM. 1986, 29(3), 315-317 | 1,15,16 | C 07 D 333/20<br>C 07 D 307/52<br>A 61 K 31/38<br>A 61 K 31/34 |
| Y | CHEMICAL ABSTRACTS, vol. 104, no. 9, 3rd March 1986, page 57, abstract no. 61899n, Columbus, Ohio, US; J.W. BANNING et al.: "Histamine receptor activation by unsaturated (allyl and propargyl) homologsof histamine", & AGENTS ACTIONS 1985, 17(2), 138-144 | 1,15,16 | |
| Y | E. SCHRÖDER et al.: "Arzneimittelchemie I", chapter 7.1, 1976, pages 24-38, Georg Thieme Verlag, Stuttgart; * pages 31-33, especially page 33, table 8, paragraph below, table 8 * | 1,15,16 | **TECHNICAL FIELDS SEARCHED (Int. Cl.4)** |
| A | EP-A-0 186 915 (MERRELL DOW PHARMACEUTICALS INC.) * claims 1, 4-7 * | 1,14-18 | C 07 D 307/00<br>C 07 D 333/00 |
| A | EP-A-0 187 390 (MERREL DOW PHARMACEUTICALS INC.) * claims 1, 4-7 * | 1,14-18 | |
| A | DE-A-2 602 846 (PARCOR) * claims 1, 4 * | 14 | |

The present search report has been drawn up for all claims

| Place of search | Date of completion of the search | Examiner |
|---|---|---|
| BERLIN | 14-05-1988 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document